# FASCICULE DE BREVET EUROPEEN

(11) **EP 0 912 536 B1**
(45) Date de publication et mention de la délivrance du brevet: **05.06.2002**
(21) Numéro de dépôt: 97931868.0
(22) Date de dépôt: 04.07.1997
(51) Int. Cl.: C07D 305/14

(54) **PROCEDE DE MONOACYLATION D'HYDROXY TAXANES**
VERFAHREN ZUR MONOACYLIERUNG VON HYDROXYTAXANEN
TAXANE HYDROXY MONOACYLATION METHOD

(30) Priorité: 09.07.1996 FR 9608505
(43) Date de publication de la demande: 06.05.1999
(73) Titulaire: Aventis Pharma S.A., 92160 Antony (FR)
(72) Inventeur: DIDIER, Eric, F-75013 Paris (FR); PECQUET, Pascal, F-27530 Ezy sur Eure (FR)
(74) Mandataire: Le Pennec, Magali
(86) Numéro de dépôt international: FR9701201
(87) Numéro de publication internationale: WO9801435

(56) Documents cités:
- F.GUERITTE-VOEGELEIN: "CHEMICAL STUDIES OF 10-DEACETYL BACCATIN III." TETRAHEDRON, (INCL TETRAHEDRON REPORTS), vol. 42, no. 16, 1986, OXFORD GB, pages 4451-4459, XP002025601 cité dans la demande

## Description

La présente invention concerne un procédé de monoacylation d'hydroxy taxanes. Elle concerne plus particulièrement un procédé de monoacylation sélective de la désacétyl-10 baccatine III ou de ses dérivés.

La désacétyl-10 baccatine III (10-DAB), représentée par la formule générale (I) dans laquelle R₁ représente un atome d'hydrogène et la baccatine III représentée par la formule générale (I) dans laquelle R₁ représente un radical acétyle, peuvent être extraites des feuilles d'ifs et constituent des matières premières intéressantes dans l'hémisynthèse du paclitaxel (Taxol®), du docetaxel (Taxotère®) et de leur dérivés. 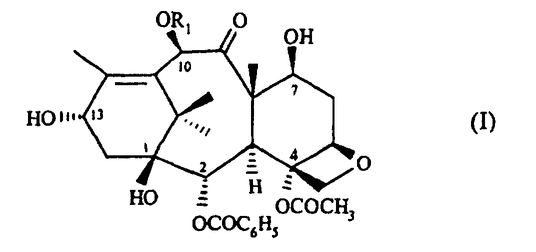 Cependant, la 10-DAB est plus facilement accessible, et est extraite des feuilles en plus grande quantité que la baccatine III.

Il est connu, d'après les travaux de F. Guéritte-Voegelein et al., Tetrahedron, 42, 4451-4460 (1986), que l'acétylation de la 10-DAB avec l'anhydride acétique est peu sélective. Lorsque la réaction a lieu dans des conditions opératoires douces (24 heures à 20°C) il se forme un mélange équimolaire de dérivés 7-monoacétylé et de 7,10-diacétylé. Des conditions plus vigoureuses (48 heures à 60°C) conduisent à la formation de 7,10-diacétylé et de 7,10,13-triacétylé en quantités égales. A une température plus élevée (24 heures à 80°C), il se forme exclusivement le dérivé 7,10,13-triacétylé.

Il est encore connu selon J-N. Denis et A. E. Greene, J. Am. Chem. Soc. 110, 5917- 5919 (1988) que la 10-DAB peut être acétylée en position 10 par le chlorure d'acétyle à condition de protéger au préalable et de façon sélective, l'hydroxyle en 7 avec un groupe triéthylsilyle.

Dans la demande internationale PCT WO 95/26961 est décrite la préparation de la baccatine III à partir de la 10-DAB en trois étapes : protection de l'hydroxyle en 7 par action d'un halogénure de trialkylsilyle, acétylation de l'hydroxyle en 10 par l'anhydride acétique, et enfin déprotection du groupe trialkylsilyle en 7 au moyen d'acide trifluoroacétique.

En tenant compte de ces résultats, il s'ensuit que toute réaction permettant la différenciation entre les hydroxyles libres en positions 7 et 10 de la 10-DAB ou de ses dérivés, notamment l'acylation sélective, est de grande importance dans la synthèse de taxoïdes.

La présente invention décrit, pour la première fois, une réaction de monoacylation sélective de l'hydroxyle en position C-10 de la désacétyl-10 baccatine III ou de ses dérivés en C-2 et/ou en C-4.

Il est connu d'après W. Kantlehner, Adv. Org. Chem., vol. 9, part 2, 65-141 et 181-277 (1976), que les halogénométhylène iminiums peuvent réagir avec les alcools pour donner les halogénures d'alkoxyméthylène iminium intermédiaires, qui conduisent après hydrolyse aux esters correspondants avec élimination d'une alkylamine.

Il a maintenant été trouvé, que la désacétyl-10 baccatine III ou ses dérivés en C-2 et/ou en C-4, peuvent être monoacylés sélectivement en position 10 en utilisant un sel de méthylène iminium répondant à la formule générale (II) : 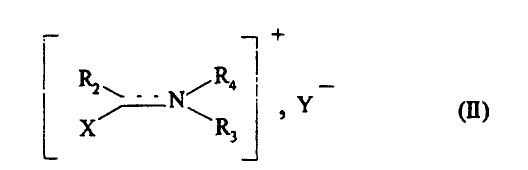 dans laquelle R₂ représente :
- un atome d'hydrogène,
- un radical alkyle droit, ramifié ou cyclique contenant 1 à 12 atomes de carbone,
- un radical alkényle droit, ramifié ou cyclique contenant 2 à 12 atomes de carbone,
- un radical alkynyle droit ou ramifié contenant 3 à 12 atomes de carbone,
- un radical aryle,
- un groupe alkoxy, alkylamino, alkylthio, alkyloxy carbonyle, alkylaminocarbonyle ou alkylthiocarbonyle dont la partie alkyle contient 1 à 12 atomes de carbone, ou
- un radical hétérocyclique, saturé ou insaturé, contenant 5 à 6 chaînons et un ou plusieurs hétéroatomes choisis parmi le soufre, l'oxygène ou l'azote,
ces radicaux étant éventuellement substitués par un ou plusieurs substituants choisis parmi:
- les atomes d'halogène,
- les radicaux alkyles ou halogénoalkyles,
- les radicaux aryles,
- les groupes alkylamino, pipéridyle, pipérazinyle, nitro ou cyano,
- les groupes alkoxy ou alkoxycarbonyles,
les parties alkyles des différents radicaux contenant 1 à 12 atomes de carbone, et
- les radicaux hétérocycliques, saturés ou insaturés, contenant 5 à 6 chaînons et un ou plusieurs hétéroatomes choisis parmi le soufre, l'oxygène ou l'azote, ou bien
R₂ et R₃ ou R₂ et R₄ peuvent former avec l'atome d'azote et le carbone méthylénique, un cycle comportant 4 à 7 chaînons, tels que la 2-pipéridone, 2-azétidinone, la 2-pyrrolidinone, le caprolactame.
R₃ et R₄, identiques ou différents, représentent chacun :
- un atome d'hydrogène,
- un radical alkyle droit, ramifié ou cyclique contenant 1 à 12 atomes de carbone,
- un radical alkényle droit, ramifié ou cyclique contenant 2 à 12 atomes de carbone,
- un radical alkynyle droit ou ramifié contenant 3 à 12 atomes de carbone, ou
- un radical aryle, ou bien
R₃ et R₄ peuvent former avec l'atome d'azote un hétérocycle contenant 4 à 6 chaînons, saturé ou insaturé, éventuellement substitué,
X représente un atome d'halogène ou un groupe phosphinyloxy, phosphoranyloxy, halogénosulfinyloxy, halogénosulfonyloxy, alkylsulfonyloxy, arylsulfonyloxy, alkylaminosulfonyloxy, alkylcarbonyloxy, arylcarbonyloxy, halophosphinyloxy, halophosphoranyloxy, haloalkylsulfonyloxy, haloalkylcarbonate,
Y⁻ représente un ion séléctionné parmi les halogénures, les alkylsulfonates, les arylsulfonates, les alkylaminosulfonates, les alkylcarboxylates, les arylcarboxylates et les phosphorodihalogénidates, ou [M(Z)ₙ]⁻ dans lequel 4 ≤ n ≤ 6, Z est un atome d'halogène et M est un élément du tableau périodique pouvant avoir un degré d'oxydation égal ou supérieur à 3 et de préférence compris entre 3 et 5.

Plus particulièrement,
X représente :
- un atome d'halogène tel qu'un atome de fluor, de chlore, de brome ou d'iode,
- un groupe (Hal)₄P-O-, (Hal)₂PO-O-, (Hal)SO-O-, (Hal)SO₂-O-, RₐSO₂-O- ou RₐCO₂ dans lesquels Rₐ représente un radical alkyle, linéaire, ramifié ou cyclique, un radical halogénoalkyle, un radical aryle éventuellement substitué par un atome d'halogène ou par un radical alkyle ou nitro, et Hal représente un atome d'halogène choisi parmi les atomes de fluor, de chlore, de brome ou d'iode,
Y⁻ représente :
- un ion halogénure tel qu'un ion fluorure, chlorure, bromure ou iodure,
- un contre-ion tel que RₐCO₂⁻, RₐSO₃⁻ ou (Hal)₂PO₂⁻, dans lequel Hal et Rₐ sont définis comme ci-dessus, ou
- une entité telle que [M(Z)ₙ]⁻ dans laquelle 4 ≤ n ≤ 6, Z est un atome d'halogène tel qu'un atome de fluor ou de chlore, et M est un élément du tableau périodique pouvant avoir un degré d'oxydation égal ou supérieur à 3, sélectionné parmi l'aluminium, le bore, l'antimoine, l'étain et le titane.

Plus particulièrement, dans la formule générale (II), R₂ peut représenter un atome d'hydrogène ou un radical méthyle, éthyle ou un radical phényle.

A titre d'exemple les sels suivants peuvent être utilisés :
le trifluorométhanesulfonate de N,N-diméthyl trifluorométhanesulfonyloxy-1 méthylidène-ammonium : (R₂=H, R₃=R₄=CH₃, X=CF₃SO₃, Y⁻=CF₃SO₃⁻) 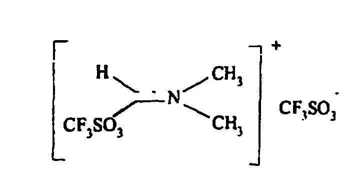
le trifluorométhanesulfonate de N-méthyl trifluorométhanesulfonyloxy-1 éthylidène-ammonium: (R₂=R₃=CH₃, R₄=H, X=CF₃SO₃, Y⁻=CF₃SO₃⁻) 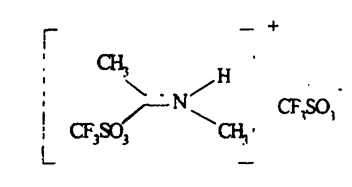
le chlorure de N-méthyl p-toluènesulfonyloxy-1 élylidène-ammonium : (R₂=R₃=CH₃, R₄=H, X=p-CH₃-C₆H₄-SO₃, Y⁻=Cl⁻) ou
le p-toluènesulfonate de N-méthyl chloro-1 éthylidène-ammonium : (R₂=R₃=CH₃, R₄=H, X=Cl, Y⁻ = p-CH₃-C₆H₄-SO₃⁻) 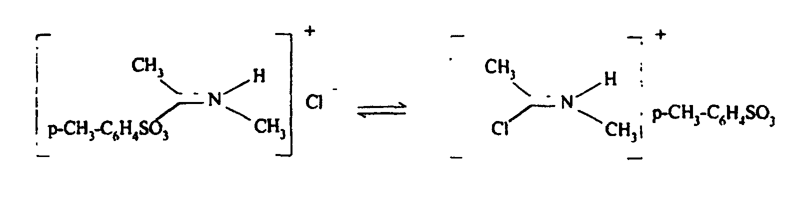
le chlorure de N-méthyl chloro-1 éthylidène-ammonium : (R₂=R₃=CH₃, R₄=H, X=Cl, Y⁻=Cl⁻) 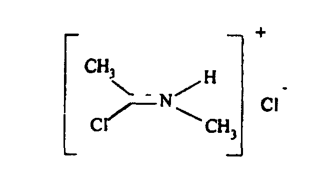
le chlorure de N-éthyl méthanesulfonyloxy-1 éthylidène-ammonium : (R₂=CH₃, R₃=C₂H₅, R₄=H, X=CH₃SO₃, Y⁻=Cl⁻)
   ou
le méthanesulfonate de N-éthyl chloro-1 élylidène-ammonium : (R₂=CH₃, R₃=C₂H₅, R₄=H, X=Cl, Y⁻= CH₃SO₃⁻) 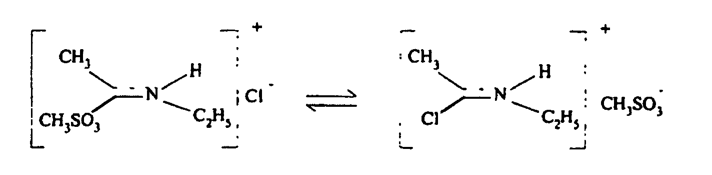
le chlorure de N-éthyl dichlorophosphinyloxy-1 éthylidène-ammonium : (R₂=CH₃, R₃=C₂H₅, R₄=H, X=O-PO-Cl₂, Y⁻=Cl⁻)
   ou
le dichlorophosphoridate de N-éthyl chloro-1 éthylidène-ammonium : (R₂=CH₃, R₃=C₂H₅, R₄=H, X=Cl, Y⁻=Cl₂PO₂⁻) 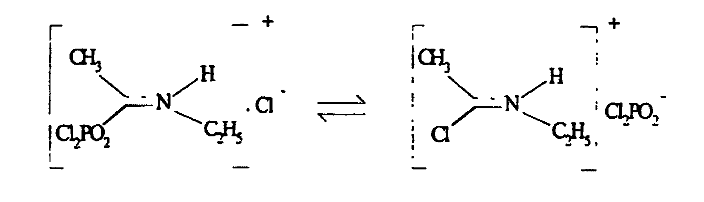
le p-toluènesulfonate de N,N-diéthyl p-toluènesulfonyloxy-1 éthylidène-ammonium : (R₂=CH₃, R₃=R₄=C₂H₅, X= p-CH₃-C₆H₄-SO₃, Y⁻= p-CH₃-C₆H₄-SO₃⁻)

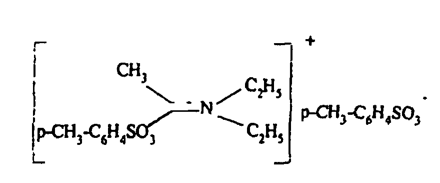

Ces sels peuvent être préparés selon des méthodes décrites dans la littérature ou en s'inspirant de celles-ci [voir par exemple W. Kantlehner, Adv. Org. Chem., vol. 9, part 2, 5-64 et 65-141 (1976), R. L. N. Harris, Synthesis, 841-842 (1980), H. S. Mosher et al., Synthetic Commun., 11, 733-736 (1981), A. G. Martinez et al., J. Chem. Soc., Chem. Commun., 1571-1572 (1990), H. Heaney et al., Tetrahedron, 49, 4015-4034 (1993), et P. L. Fuchs et al. J. Org. Chem., 59, 348-354 (1994)].

Les sels de méthylène iminiums (II) sont préparés par mise en contact d'une amide de formule 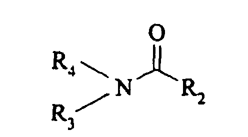 avec un réactif électrophile de formule XY. Ils peuvent être isolés ou formés « in situ ». L'amide peut être utilisée à la fois comme réactif et comme solvant réactionnel.

Selon l'invention, les sels de méthylène iminiums peuvent réagir avec la 10-DAB et/ou ses dérivés en C-2 et C-4 de formule générale (III) : 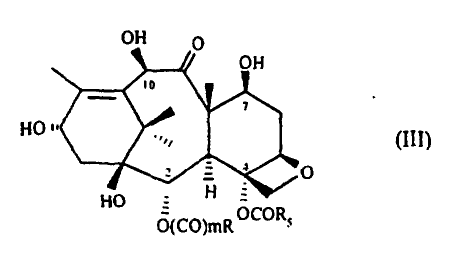 dans laquelle m peut être égal à 0 ou 1,
R et R₅, identiques ou différents représentent :
- un radical alkyle droit, ramifié ou cyclique contenant 1 à 8 atomes de carbone,
- un radical alkényle droit, ramifié ou cyclique contenant 2 à 8 atomes de carbone,
- un radical alkynyle droit, ramifié ou cyclique contenant 3 à 8 atomes de carbone,
- un radical aryle, ou
- un radical hétérocyclique, saturé ou insaturé, contenant 4 à 6 chaînons avec un ou plusieurs hétéroatomes choisis parmi les atomes d'oxygène, d'azote ou du soufre,
ces radicaux étant éventuellement substitués par un ou plusieurs substituants choisis parmi :
- les atomes d'halogène tel que les atomes de fluor, de chlore, de brome ou d'iode,
- les radicaux alkyles, hydroxy, alkoxy, alkylthio, alkylamino, halogénoalkyles, halogénoalkoxy, halogénoalkylthio dont la chaîne alkyle contient 1 à 4 atomes de carbone, ou
- les radicaux hétérocycliques ayant de 4 à 6 chaînons comportant un ou plusieurs hétéroatomes choisi parmi les atomes d'oxygène, d'azote ou du soufre, éventuellement substitués,
- les radicaux aryles éventuellement substitués,
- les groupes cyano, nitro ou azido, ou
- les groupes carboxy ou alkoxycarbonyles dont la partie alkyle contient 1 à 4 atomes de carbone,
pour fournir un produit de formule générale (V) : 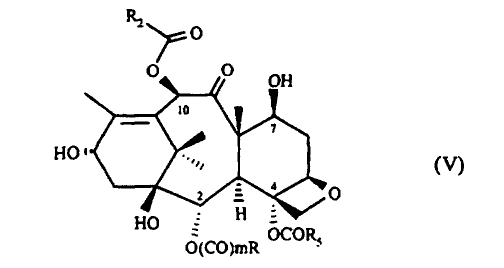 dans laquelle R, R₂ et R₅ sont définis comme précédemment, en passant intermédiairement par un produit de formule générale (IV) : 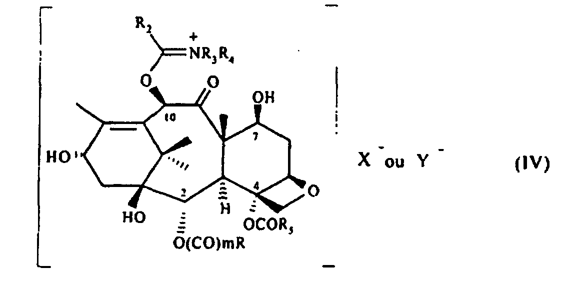 dans laquelle m, R, R₂, R₃, R₄, R₅, X⁻ et Y⁻ sont définis comme précédemment qui est hydrolysé en produit de formule générale (V).

Les composés de formule générale (III) sont définis selon l'enseignement des demandes internationales PCT WO 94/20484 et WO 95/33736.

Selon une meilleure manière de mise en oeuvre de l'invention on utilise un excès de réactif électrophile et/ou d'amide par rapport au substrat de formule (III). Selon un mode préférentiel, l'excès de réactif électrophile est inférieur ou égal à 10 équivalents et encore plus préférentiellement voisin de 5 équivalents par rapport au réactif de formule générale (III).

Généralement, la réaction est mise en oeuvre dans un solvant organique choisi parmi les hydrocarbures aliphatiques halogénés, de préférence les hydrocarbures aliphatiques chlorés tels que le dichlorométhane ou le dichloro-1,2-éthane, les hydrocarbures aromatiques tels que le benzène, le toluène ou le xylène, les éthers aliphatiques tels que l'éther éthylique ou l'éther isopropylique, les nitriles aliphatiques tels que l'acétonitrile ou les amides aliphatiques tels que le N,N-diméthylformamide, le N,N-diméthylacétamide, le N-éthylacétamide et le N,N-diéthylacétamide. Il est particulièrement avantageux d'effectuer la réaction dans l'amide aliphatique qui est utilisé pour la préparation des sels de méthylène iminiums (II).

Généralement, le procédé selon l'invention est mis en oeuvre à une température comprise entre -78°C et la température de reflux du mélange réactionnel et de préférence entre -20°C et 25°C.

Il peut être avantageux de mettre en oeuvre le procédé en présence d'une base minérale ou organique choisie parmi la pyridine, les trialkylamines, les N-alkylmorpholines, les carbonates ou les hydrogénocarbonates de métaux alcalins.

L'hydrolyse des sels d'iminium de formule générale (IV) en produit de formule générale (V) peut être effectuée à l'eau ou au moyen d'une solution aqueuse d'une base minérale ou organique telle que la soude, la pyridine, la triéthylamine, l'acétate de sodium, le carbonate de sodium ou l'hydrogénocarbonate de sodium.

Les composés de formule générale (V) sont particulièrement utiles pour la préparation des composés de formule générale (VI) : 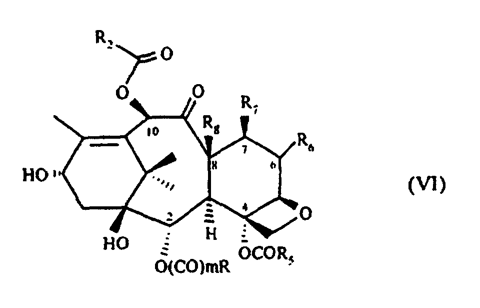 dans laquelle,
m, R, R₂ et R₅ sont définis comme précédemment,
R₆ représente un atome d'hydrogène, ou forme une liaison avec R₇ conduisant ainsi à une double liaison entre C-6 et C-7,
R₇ représente :
- un atome d'hydrogène,
- un atome d'halogène, ou
- un groupe hydroxy, ORₓ, ou O-CORₓ dans lesquels Rₓ représente un atome d'hydrogène ou un radical alkyle droit, ramifié ou cyclique contenant 1 à 8 atomes de carbone, un radical alkényle droit, ramifié ou cyclique contenant 2 à 8 atomes de carbone, un radical alkynyle droit ou ramifié contenant 3 à 8 atomes de carbone, ou un radical aryle, étant entendu que ces radicaux peuvent être éventuellement substitués, ou
- un groupe augmentant l'hydrosolubilité de formule générale -O-CO-A-COR_{y},
dans laquelle A est un radical alkényle, cycloalkényle ou aryle comportant au moins une double liaison,
R_{y} représente :
- un groupe hydroxyle,
- un groupe alkylamino, alkylaminoalkyloxy, alkylaminoalkylthio, N,N-dialkylaminocarbonylalkoxy contenant 1 à 4 atomes de carbone éventuellement substitué, et
R₈ représente un radical méthyle ou forme un cyclopropyle avec R₇.

La baccatine III de formule générale (V) obtenue selon la présente invention est utilisée, en particulier, pour la préparation de produits de formule générale (VI) dans laquelle R₂ représente un radical méthyle, R₆ représente un atome hydrogène, R₈ et R₇ forme un cyclopropyle, m est égal à 1, R représente un radical phényle et R₅ représente un radical méthyle, soit par action de l'anhydride trifluorométhanesulfonique, puis d'un halogénure de métal alcalin (chlorure de sodium, iodure de sodium, fluorure de potassium), ou avec l'azoture de métal alcalin (azoture de sodium), ou avec le sel d'ammonium quaternaire ou avec un phosphate de métal alcalin, soit par action du DAST ("diethylaminosulfur trifluoride")

Il est particulièrement avantageux d'utiliser la baccatine III ou ses dérivés de formule générale (VI) obtenus selon le procédé de la présente invention, pour la préparation des taxoïdes thérapeutiquement actifs, dont la fonction hydroxyle en 7 est éventuellement modifiée, de formule générale (VII): 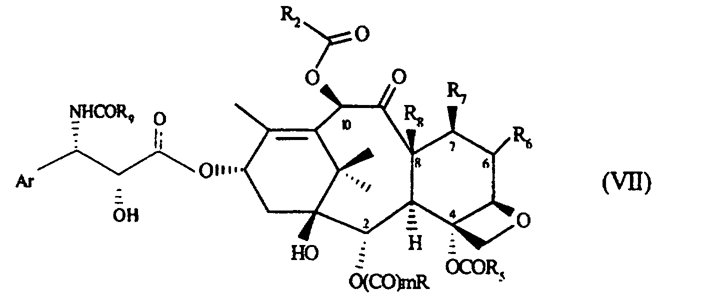 dans laquelle m est égal à 1,
R représente un radical phényle,
R₂, R₅ représentent un radical méthyle,
R₆ représente un atome d'hydrogène,
R₇ représente des substituants tels que définis précédemment,
R₈ peut représenter un radical méthyle ou former avec R₇ un cyclopropyle, et
de préférence Ar représente un radical phényle et R₉ représente un radical t-butoxy ou phényle.

Plus particulièrement, la baccatine III ou ses dérivés en C-7, préparés selon le procédé de la présente invention, peuvent être utilisés pour la synthèse du paclitaxel (Taxol®) ou du dérivé de formule générale (VII) dans laquelle m est égal à 1, R représente un radical phényle, R₂ et R₅ représentent chacun un radical méthyle, R₆ représente un atome d'hydrogène, R₈ et R₇ forment un cyclopropyle, Ar représente un radical phényle et R₉ représente un radical t-butoxy comme décrit dans la demande internationale WO 94/13654.

Les exemples suivants illustrent la présente invention.

### EXEMPLE 1

### Préparation du 4-acétoxy-2α-benzoyloxy-5β,20-époxy-10β-formyloxy-1,7β,13α-trihydroxy-9 oxo-tax-11-ène

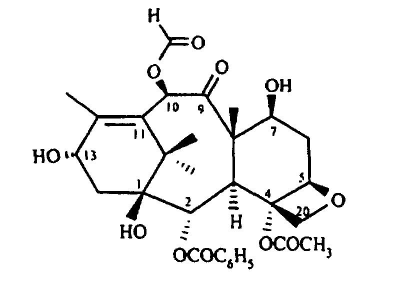

Dans un monocol de 50 ml, muni d'un thermomètre et d'une agitation magnétique, on charge sous atmosphère d'azote 12 ml de N,N-diméthylformamide (155 mmol), 0,495 ml de pyridine (6,1 mmol) et 3 g de 4-acétoxy-2α-benzoyloxy-5β ,20-époxy-1,7β,10β,13α-tétrahydroxy-9-oxo-tax-11-ène (10-DAB) à 97,7% (5,4 mmol). Après refroidissement à -20°C, on ajoute en 10 minutes par l'intermédiaire d'une seringue 1,08 ml d'anhydride triflique (6,4 mmol). Le mélange réactionnel est maintenu pendant 10 minutes à -20°C puis on hydrolyse avec 60 ml d'eau. Après 3 heures à 0°C, la suspension est filtrée et le produit obtenu est séché sous pression réduite à température ambiante pendant une nuit. On obtient ainsi, avec un rendement d'environ 90%, 2,87 g (4,9 mmoles) de 4-acétoxy-2α-benzoyloxy-5β ,20-époxy-10β-formyloxy-1,7β,13α-trihydroxy-9-oxo-tax-11-ène dont la pureté déterminée par CLHP est de 97%, et dont les caractéristiques sont les suivantes :
- spectre de RMN ¹H (400 MHz, CDCl₃, δ en ppm) : 1,11 (s, 3H, CH₃) ; 1,13 (s, 3H, CH₃) ; 1,69 (s, 1H, OH-1) ; 1,71 (s, 3H, CH₃) ; 1,87 et 2,58 (2 m, 1H chacun, CH₂-6) ; 2,08 (s, 3H, CH₃) ; 2,18 (d, J = 5,0 Hz, 1H, OH-13) ; 2,25 (d, J = 5,0 Hz, 1H, OH-7) ; 2,29 (s, 3H, COOCH₃); 2,32 (d, J = 9,0 Hz, 2H, CH₂-14) ; 3,91 (d, J = 7,0 Hz, 1H, H-3) ; 4,16 et 4,33 (2d, J = 8,5 Hz, 1H chacun, CH₂-20) ; 4,47 (m, 1H, H-7) ; 4,91 (m, 1H, H-13) ; 5.00 (d large, J = 10,0 Hz, 1H, H-5) ; 5,64 (d, J = 7,0 Hz, 1H, H-2) ; 6,46 (s, 1H, H-10) ; 7,50 (t, J = 7,5 Hz, 2H, H-méta dans C₆H₅COO) ; 7,64 (t, J = 7,5 Hz, 1H, H-para dans C₆H₅CO) ; 8,13 (d, J = 7,5 Hz, 2H, H-ortho dans C₆H₅CO) ; 8;23 (s, 1H, HCO).
- spectre IR (KBr, ν en cm⁻¹) : 3622 et 3517 (OH de H₂O) ; 3400 (OH alcools + H₂O) ; 3061 et 3019 (CH aromatiques) ; 2990 à 2850 (CH₂, CH₃) ; 1739 (C=O acétate) ; 1715 (C=O formiate + cétone) ; 1704 (C=O benzoate) ; 1271 (O-C=O benzoate) ; 1250 (O-C=O acétate) ; 1156 (O-C=O formiate) ; 1100 à 1000 (C-O alcools) ; 977 (C-O oxétane) ; 719 (CH aromatiques).
- spectre de masse (NH₃) M/Z: 590 (M+NH₄⁺); 573 (M+H⁺); 544 (M-CHO+NH₄⁺); 527 (544-OH).

### EXEMPLE 2

### Préparation du 4,10β-diacétoxy-2α-benzoyloxy-5β,20-époxy-1,7β,13α-trihydroxy-9-oxo-tax-11-ène (baccatine III)

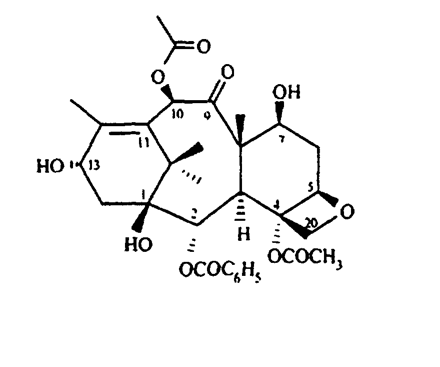

Dans un réacteur double-enveloppe de 250 ml, muni d'un thermomètre et d'une agitation magnétique, on charge sous azote 60 ml de N-éthylacétamide (627 mmol). Après refroidissement à -20°C, on ajoute en 15 minutes par l'intermédiaire d'une seringue 7,2 ml de chlorure de mésyle (92 mmol). La solution est maintenue pendant 30 minutes à -20°C puis pendant environ 17 heures à 0°C. On ajoute à la solution, à 0°C, 10 g (10,7 mmoles) de 4-acétoxy-2α-benzoyloxy-5β,20-époxy-1,7β,10β,13α-tétrahydroxy-9-oxo-tax-11-ène (10-DAB) à 97,5 % puis la suspension est maintenue sous agitation pendant 30 heures à 0°C. Après transfert dans un réacteur de 1 litre, on ajoute dans le mélange réactionnel, une solution de 15 g d'acétate de sodium dans 300 ml d'eau puis la suspension obtenue est maintenue pendant 25 heures à température ambiante. L'analyse par CLHP d'un aliquot du mélange réactionnel permet de détecter moins de 1% des dérivés monoacétylé en 7 et diacétylé en 7,10. Après filtration, le produit est lavé successivement trois fois avec 50 ml d'eau et séché une nuit à 50°C sous pression réduite. On obtient ainsi, avec un rendement de 79 %, 9 g (14,1 mmoles) de 4,10β-diacétoxy-2α-benzoyloxy-5β,20-époxy-1,7β,13α-trihydroxy-9-oxo-tax-11-ène dont la pureté est de 92%, et dont les caractéristiques sont les suivantes :
- spectre de RMN ¹H (400 MHz, CDCl₃, δ en ppm) : 1,12 (s, 6H, CH₃) ; 1,68 (s, 4H, CH₃ et OH-1) ; 1,88 et 2,58 (2 m, 1H chacun, CH₂-6) ; 2,08 (s, 3H, CH₃) ; 2,27 et 2,31 (2 s, 3H chacun, CH₃CO et CH₃COO) ; 2,32 ( d, J = 9,0 Hz, 2H, CH₂-14) ; 3,90 ( d, J = 7,0 Hz, 1H, H-3) ; 4,17 et 4,33 (2 d, J = 8,5 Hz, 1H chacun, CH₂-20) ; 4,48 (dd, J = 11,0 Hz et 7,0 Hz, 1H, H-7) ; 4,91 (t large, J = 9,0 Hz , 1H, H-13) ; 5,00 (d large, J= 10,0 Hz, 1H, H-5) ; 5,64 (d, J = 7,0 Hz, 1H, H-2) ; 6,34 (s, 1H, H-10) ; 7,50 (t, J = 7,5 Hz, 2H, H-méta dans C₆H₅COO) ; 7,64 (t, J = 7,5 Hz, 1H, H-para dans C₆H₅COO) ; 8,13 (d, J = 7,5 Hz, 2H, H-ortho dans C₆H₅COO).
- spectre IR (KBr, v en cm⁻¹): 3400 (OH alcools + H₂O) ; 3071 (CH aromatiques) ; 2990 à 2850 (CH₂ et CH₃) ; 1730 (C=O acétate) ; 1712 (C=O formiate + cétone + benzoate) ; 1271 (O-C=O benzoate) ; 1243 (O-C=O acétate) ; 1100 à 1000 (C-O alcools); 982 (C-O oxétane) ; 710 (CH aromatiques).
- spectre de masse (NH₃) M/Z: 604 (M+NH₄⁺); 587 (M+H⁺); 544 (M-CH₃COOH+NH₄⁺); 527 (M+CH₃COOH+H⁺); 509 (M-CH₃COOH-H₂O+H⁺).

### EXEMPLE 3

Dans un erlenmeyer de 25 ml, muni d'une agitation magnétique, on charge sous azote 3,9 g de N-méthylacétamide (53 mmol) et 5 ml de 1,2-dichloroéthane. Après refroidissement à -10°C, on ajoute 0,42 ml d'anhydride triflique (2,5 mmol) puis la solution est maintenue pendant 30 minutes à -10°C puis pendant 1 heure à 0°C. On ajoute à la solution, à 0°C, 0,5 g (environ 0,9 mmol) de 4-acètoxy-2α-benzoyloxy-5β, 20-époxy-1,7β,10β,13α-tétrahydroxy-9-oxo-tax-11-ène (10-DAB). Le mélange réactionnel résultant est maintenu sous agitation pendant une nuit à 0°C. On ajoute alors 15 ml d'une solution aqueuse d'acétate de sodium à 5% et puis maintient une nuit à température ambiante. Le mélange réactionnel est dilué par addition de 1,2-dichloroéthane jusqu'à dissolution complète. La phase organique est séparée par décantation puis le solvant est évaporé sous pression réduite. Dans l'extrait obtenu, on dose par CLHP environ 33mg de 4,10β-diacetoxy-2α-benzoyloxy-5β,20-époxy-1,7β ,13α-trihydroxy-9-oxo-tax-11-ène (baccatine III). Le rendement est voisin de 6 %.

### EXEMPLE 4

Dans un erlenmeyer de 25 ml, muni d'une agitation magnétique, on charge sous azote à température ambiante 5 ml d'acétonitrile, 3,9 g de N-méthylacétamide (53 mmol) et 0,9g de chlorure de tosyle (4,7 mmol) puis la solution est maintenue pendant 20 minutes à température ambiante. On ajoute à la solution obtenue 0,5 g (environ 0,9 mmol) de 4-acétoxy-2α-benzoyloxy-5β,20-époxy-1,7β,10β, 13α-tétrahydroxy-9-oxo-tax-11-ène (10-DAB), puis le mélange réactionnel est maintenu sous agitation pendant environ 17 heures à température ambiante. On ajoute alors 15 ml d'une solution aqueuse d'acétate de sodium à 5% puis on maintient sous agitation pendant une nuit à température ambiante. L'analyse CLHP d'un aliquot ne permet pas de détecter de dérivés monoacétylé en 7 ou de diacétylé en 7,10 par. Le mélange réactionnel est extrait avec du 1,2-dichloroéthane, la phase organique est séparée après décantation et le solvant est évaporé sous pression réduite. Dans l'extrait obtenu, on dose par CLHP environ 86 mg de 4,10β-diacétoxy-2α-benzoyloxy-5β,20-époxy-1,7β,13α-trihydroxy-9-oxo-tax-11-ène (baccatine III), soit un rendement d'environ 16%.

### EXEMPLE 5

Dans un erlenmeyer de 25 ml, muni d'une agitation magnétique, on charge sous azote à température ambiante 5 ml de 1,2-dichloroéthane, 3,9g de N-méthylacétamide (53 mmol). Après refroidissement à 0°C, on coule 0,4 ml de chlorure d'oxalyle (4,6 mmol) puis la solution est maintenue pendant 25 minutes à 0°C (formation de gaz). On ajoute à la solution obtenue 0,5 g (environ 0,9 mmol) de 4-acétoxy-2a-benzoyloxy-5β,20-époxy-1,7β,10β,13α-tétrahydroxy-9-oxo-tax-11-ène (10-DAB), puis le mélange réactionnel est maintenu sous agitation pendant environ 19 heures à 0°C. On ajoute alors 15 ml d'une solution aqueuse d'acétate de sodium à 5% et on maintient pendant une nuit à température ambiante. Le mélange réactionnel est extrait avec du 1,2-dichloroéthane. La phase organique est séparée par décantation et le solvant est évaporé sous pression réduite. Dans l'extrait obtenu, on dose par CLHP environ 212 mg de 4,10β-diacétoxy-2α-benzoyloxy-5β,20-époxy-1,7β,13α-trihydroxy-9-oxo-tax-11-ène (baccatine III), soit un rendement d'environ 40%. On détecte 1,2% du dérivé monoacétylé en 7. Le dérivé diacétylé en 7,10 n'est pas détecté.

### EXEMPLE 6

Dans un erlenmeyer de 50 ml, muni d'une agitation magnétique, on charge sous azote à température ambiante 5 ml de 1,2-dichloroéthane et 1,5 g d'anhydride p-toluènesulfonique (4,5 mmol). Après refroidissement à 0°C, on ajoute 4 ml de N,N-diéthylacétamide (32 mmol) puis la solution est maintenue pendant 35 minutes à 0°C. On ajoute à la suspension obtenue, 0,5 g (environ 0,9 mmol) de 4-acétoxy-2α-benzoyloxy-5β,20-époxy-1,7β,10β,13α-tétrahydroxy-9-oxo-tax-11-ène (10-DAB). Le mélange réactionnel est maintenu sous agitation pendant environ 1 heure et 30 minutes à 0°C puis 4 heures et 30 minutes à température ambiante. Un aliquot de la solution est prélevé et hydrolysé dans un mélange acétonitrile/eau (diluant CLHP). On dose ainsi par CLHP, dans la totalité de la solution, environ 347 mg de 4,10β-diacétoxy-2α-benzoyloxy-5β,20-époxy-1,7β,13α-trihydroxy-9-oxo-tax-11-ène (baccatine III), soit un rendement d'environ 66%. Aucun dérivé monoacétylé en 7 ni diacétylé en 7,10, n'est détecté.

### EXEMPLE 7

Dans un erlenmeyer de 25 ml, muni d'une agitation magnétique, on charge sous azote à température ambiante 4 ml de N-éthylacétamide (42 mmol) et 5 ml de 1,2-dichloroéthane. On ajoute 0,42 ml d'oxychlorure de phosphore (4,6 mmol). La solution est maintenue pendant 15 minutes à température ambiante. On ajoute à la solution 0,5 g (environ 0,9 mmol) de 4-acétoxy-2α-benzoyloxy-5β,20-époxy-1,7β,10β ,13α-tétrahydroxy-9-oxo-tax-11-ène (10-DAB). Le mélange réactionnel résultant est maintenu sous agitation pendant environ 3 heures. Un aliquot de la solution est prélevé et hydrolysé dans un mélange acétonitrile/eau (diluant CLHP). Dans la totalité de la solution, on dose par CLHP, environ 116 mg de 4,10β-diacétoxy-2α-benzoyloxy-5β ,20-époxy-1,7β,13α-trihydroxy-9-oxo-tax-11-ène (baccatine III) soit un rendement d'environ 22%. On ne détecte pas de dérivés monoacétylé en 7 et de diacétylé en 7,10.

## Revendications

1. Procédé de monoacylation sélective de la désacétyl-10 baccatine III ou de ses dérivés en C-2 et C-4, possédant des groupements hydroxyles libres en positions C-7 et C-10, **caractérisé en ce que** l'on effectue l'acylation de la 10-désacétylbaccatine III ou de ses dérivés en C-2 et C-4 au moyen d'un sel de méthylène iminium.

2. Procédé selon la revendication 1 **caractérisé en ce que** le sel de méthylène iminium répond à la formule générale (II) : 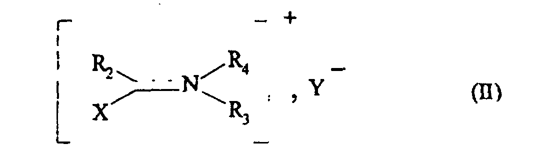 dans laquelle R₂ représente :
- un atome d'hydrogène,
- un radical alkyle droit, ramifié ou cyclique contenant 1 à 12 atomes de carbone,
- un radical alkényle droit, ramifié ou cyclique contenant 2 à 12 atomes de carbone,
- un radical alkynyle droit ou ramifié contenant 3 à 12 atomes de carbone,
- un radical aryle,
- un groupe alkoxy, alkylamino, alkylthio, alkyloxy carbonyl, alkylamino carbonyl, alkylthio carbonyl, dont la partie alkyle comprend 1 à 12 atomes de carbone, ou
- un radical hétérocyclique, saturé ou insaturé, comprenant 5 à 6 chaînons et un ou plusieurs hétéroatomes choisis parmi le soufre, l'oxygène ou l'azote,
ces radicaux étant éventuellement substitués par un ou plusieurs substituants choisis parmi :
- les atomes d'halogène,
- les radicaux alkyle ou halogénoalkyle,
- les radicaux aryles,
- les groupes alkylamino, pipéridyle, pipérazinyle, nitro ou cyano,
- les groupes alkoxy ou alkoxycarbonyle,
dont les parties alkyles contiennent 1 à 12 atomes de carbone, et
- les radicaux hétérocycliques, saturés ou insaturés, contenant 5 à 6 chaînons et un ou plusieurs hétéroatomes choisis parmi le soufre, l'oxygène ou l'azote, ou bien
R₂ et R₃ ou R₂ et R₄ peuvent former avec l'atome d'azote et le carbone méthylénique, un cycle comportant 4 à 7 chaînons,
R₃ et R₄, identiques ou différents, représentent :
- un atome d'hydrogène,
- un radical alkyle droit, ramifié ou cyclique contenant 1 à 12 atomes de carbone,
- un radical alkényle droit, ramifié ou cyclique contenant 2 à 12 atomes de carbone,
- un radical alkynyle droit ou ramifié contenant 3 à 12 atomes de carbone, ou
- un radical aryle, ou bien
R₃ et R₄ peuvent former avec l'atome d'azote un cycle contenant 4 à 6 chaînons, saturé ou insaturé, éventuellement substitué,
X représente un atome d'halogène, un groupe phosphinyloxy, phosphoranyloxy, halogénosulfinyloxy, halogénosulfonyloxy, alkylsulfonyloxy, arylsulfonyloxy, alkylaminosulfonyloxy, alkylcarbonyloxy, arylcarbonyloxy, halophosphinyloxy, halophosphoranyloxy, haloalkylsulfonyloxy, haloalkylcarbonate,
Y⁻ représente un ion séléctionné parmi les halogénures, les alkylsulfonates, les arylsulfonates, les alkylaminosulfonates, les alkylcarboxylates, les arylcarboxylates, les phosphorodihalogénidates, ou [M(Z)ₙ]⁻ dans lequel 4 ≤ n ≤ 6, Z est un atome d'halogène et M est un élément du tableau périodique pouvant atteindre un degré d'oxydation égal ou supérieur à 3 et de préférence compris entre 3 et 5.

3. Procédé selon la revendication 2 **caractérisé en ce que** dans la formule générale (II),
X représente :
- un atome d'halogène,
- un groupe (Hal)₄P-O-, (Hal)₂PO-O-, (Hal)SO-O-, (Hal)SO₂-O-, RₐSO₂-O- ou RₐCO₂ dans lesquels Rₐ représente un radical alkyle, linéaire, ramifié ou cyclique, un radical halogenoalkyle ou un radical aryle éventuellement substitué par un atome d'halogène ou par un radical alkyle ou nitro, et Hal représente un atome d'halogène choisi parmi les atomes de fluor, de chlore, de brome ou d'iode,
Y⁻ représente :
- un ion halogénure,
- un contre-ion tel que RₐCO₂⁻, RₐSO₃⁻ ou (Hal)₂PO₂⁻, dans lequel Hal et Rₐ sont définis comme ci-dessus, ou
- une entité telle que [M(Z)ₙ]⁻ dans laquelle 4 ≤ n ≤ 6, Z est un atome d'halogène tel qu'un atome de fluor ou de chlore, et M est un élément du tableau périodique pouvant avoir un degré d'oxydation égal ou supérieur à 3, sélectionné parmi l'aluminium, le bore, l'antimoine, l'étain et le titane.
R₂ représente l'hydrogène, un radical méthyle, éthyle ou un radical phényle.

4. Procédé selon l'une des revendications 1 à 3 dans lequel les sels d'iminium que l'on utilise sont le trifluorométhanesulfonate de N,N-diméthyl trifluorométhanesulfonyloxy-1 méthylidène ammonium (R₂=H, R₃=R₄=CH₃, X=CF₃SO₃, Y⁻=CF₃SO₃⁻), le trifluorométhanesulfonate de N-méthyl trifluorométhanesulfonyloxy-1 éthylidène-ammonium (R₂=R₃=CH₃, R₄=H, X=CF₃SO₃, Y⁻=CF₃SO₃⁻), le chlorure de N-méthyl p-toluènesulfonyloxy-1 éthylidène-ammonium (R₂=R₃=CH₃, R₄=H, X=p-CH₃-C₆H₄-SO₃, Y⁻=Cl⁻) ou le p-toluènesulfonate de N-méthyl chloro-1 éthylidène-ammonium (R₂=R₃=CH₃, R₄=H, X=Cl, Y⁻= p-CH₃-C₆H₄-SO₃⁻), le chlorure de N-méthyl chloro-1 éthylidène-ammonium (R₂=R₃=CH₃, R₄=H, X=Cl, Y⁻=Cl⁻), le chlorure de N-éthyl méthanesulfonyloxy-1 éthylidène-ammonium (R₂=CH₃, R₃=C₂H₅, R₄=H, X=CH₃SO₃, Y⁻= Cl⁻) ou le méthanesulfonate de N-éthyl chloro-1 éthylidène-ammonium (R₂=CH₃, R₃=C₂H₅, R₄=H, X=Cl, Y⁻= CH₃SO₃⁻), le chlorure de N-éthyl dichlorophosphinyloxy-1 éthylidène-ammonium (R₂=CH₃, R₃=C₂H₅, R₄=H, X=O-PO-Cl₂, Y⁻=Cl⁻) ou le dichlorophosphoridate de N-éthyl chloro-1 éthylidène-ammonium, (R₂=CH₃, R₃=C₂H₅, R₄=H, X=Cl, Y⁻=Cl₂PO₂⁻), le p-toluènesulfonate de N,N-diéthyl p-toluènesulfonyloxy-1 éthylidène-ammonium (R₂=CH₃, R₃=R₄=C₂H₅, X= p-CH₃-C₆H₄-SO₃, Y⁻= p-CH₃-C₆H₄-SO₃⁻).

5. Procédé selon la revendication 1 **caractérisé en ce que** la désacétyl-10 baccatine III et/ou ses dérivés en C-2 et C-4 répondent à la formule générale (III) : 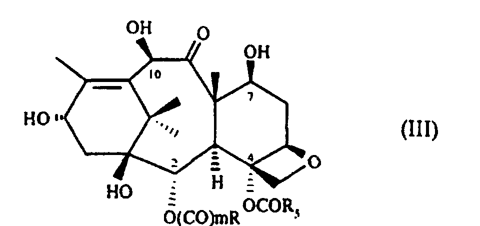 dans laquelle m est égal à 0 ou 1,
R et R₅, identiques ou différents, représentent :
- un radical alkyle droit, ramifié ou cyclique contenant 1 à 8 atomes de carbone,
- un radical alkényle droit, ramifié ou cyclique contenant 2 à 8 atomes de carbone,
- un radical alkynyle droit, ramifié ou cyclique contenant 3 à 8 atomes de carbone,
- un radical aryle, ou
- un radical bétérocyclique, saturé ou insaturé, contenant 4 à 6 chaînons avec un ou plusieurs hétéroatomes choisis parmi les atomes d'oxygène, d'azote ou du soufre,
ces radicaux étant éventuellement substitués par un ou plusieurs substituants choisis parmi :
- les atomes d'halogène,
- les radicaux alkyles, hydroxy, alkoxy, alkylthio, alkylamine, halogénoalkyles, halogénoalkoxy, halogénoalkylthio, dont la partie alkyle contient 1 à 4 atomes de carbone,
- les radicaux hétérocycliques ayant de 4 à 6 chaînons comportant un ou plusieurs hétéroatomes choisi parmi les atomes d'oxygène, d'azote ou du soufre, éventuellement substitués,
- les radicaux aryles éventuellement substitués,
- les groupes cyano, nitro ou azido, ou
- les groupes carboxy ou alkoxycarbonyles dont la partie alkyle contient 1 à 4 atomes de carbone.

6. Procédé selon la revendication 5 **caractérisé en ce que**, dans la formule générale (III), m est égal à 1, R représente un radical phényle et R₅ représente un radical méthyle.

7. Procédé selon la revendication 1 **caractérisé en ce que** l'on fait réagir un sel de méthylène iminium tel que défini dans l'une des revendications 2 à 5 sur un dérivé de la 10-DAB tel que défini dans l'une des revendications 6 ou 7 pour obtenir un produit de formule générale (IV) : 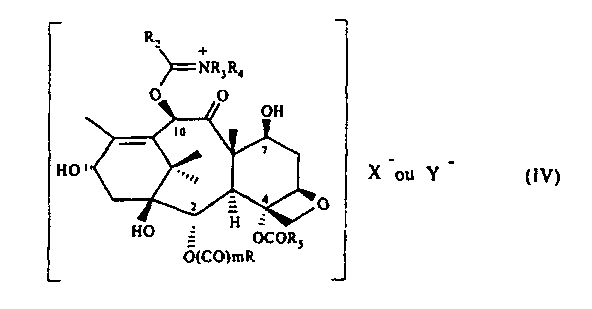 dans laquelle R₂, R₃, R₄, X⁻ et Y⁻ sont définis comme dans l'une des revendications 2 à 5 et m, R et R₅ sont définis corne dans l'une des revendications 6 ou 7, qui est hydrolysé en produit de formule générale (V) : 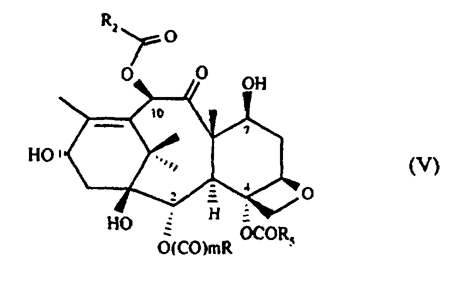 dans laquelle R₂ est défini comme dans l'une des revendications 2 à 4 et m, R et R₅ sont définis comme dans l'une des revendications 5 ou 6.

8. Procédé selon l'une des revendications 1 à 7 **caractérisé en ce que** l'on opère dans un solvant organique.

9. Procédé selon la revendication 8 **caractérisé en ce que** le solvant organique est choisi parmi les solvants organiques halogénés, les hydrocarbures aromatiques, les éthers aliphatiques, les nitriles aliphatiques ou les amides aliphatiques.

10. Procédé selon la revendication 8 **caractérisé en ce que** les solvants organiques halogénés sont choisis parmi les hydrocarbures aliphatiques chlorés tels que le dichlorométhane ou le dichloro-1,2-éthane, les hydrocarbures aromatiques sont choisis parmi le benzène, le toluène ou le xylène, les éthers aliphatiques sont choisis parmi l'éther éthylique ou l'éther isopropylique, les nitriles aliphatiques tels que l'acétonitrile, ou les amides aliphatiques sont choisis parmi le N,N-diméthylformamide, le N,N-diméthylacétamide, le N-éthylacétamide et le N,N-diéthylacétamide.

11. Procédé selon l'une quelconque des revendications précédentes **caractérisé en ce que** le sel de méthylène iminium de formule (II) est préparé par condensation de l'amide de formule 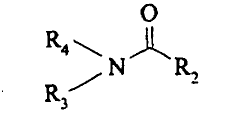 avec un réactif électrophile de formule XY.

12. Procédé selon l'une quelconque des revendications 1 à 10 **caractérisé en ce que** le sel de méthylène iminium est préparé in situ ou isolé préalablement.

13. Procédé selon les revendications 1 à 12 **caractérisé en ce que** l'on opère en présence d'un excès de réactif électrophile inférieur ou égal à 10 équivalents par rapport au substrat de formule générale (III)

14. Procédé selon la revendication 13 **caractérisé en ce que** le réactif électrophile est voisin de 5 équivalents par rapport au substrat de formule générale (III).

15. Procédé selon la revendications 1 **caractérisé en ce que** la réaction des sels de méthylène iminiums de formule générale (II) avec les substrats de formule générale (III) est mise en oeuvre à une température comprise entre -78°C et la température de reflux du mélange réactionnel.

16. Procédé selon la revendication 15 **caractérisé en ce** la température est comprise entre -20°C et 25°C.

17. Procédé selon la revendication 7 **caractérisé en ce que** l'hydrolyse des sels d'iminium de formule générale (IV) est effectuée à l'eau ou au moyen d'une solution aqueuse contenant une base inorganique ou organique.

18. Procédé selon la revendication 17 **caractérisé en ce que** la base est choisie parmi la soude, la pyridine, la triéthylamine, l'acétate de sodium, le carbonate de sodium et l'hydrogénocarbonate de sodium.

19. Nouveaux dérivés de la baccatine III de formule générale (IV) : 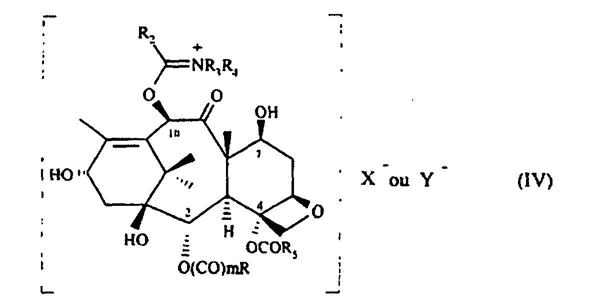 dans laquelle R₂, R₃, R₄, X⁻ et Y⁻ sont définis comme dans l'une des revendications 2 à 5, et m, R et R₅ sont définis comme dans l'une des revendications 6 ou 7.

20. Utilisation des produits obtenus selon l'une quelconque des revendications précédentes pour la préparation des analogues de la baccatine III de formule générale (VI) : 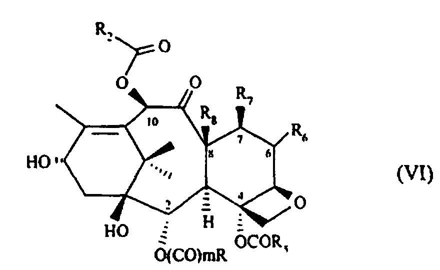 dans laquelle,
R₂ est défini comme dans l'une des revendications 2 à 4
m, R et R₅ sont définis comme dans l'une des revendications 5 ou 6
R₆ représente un atome d'hydrogène, ou forme une liaison avec R₇ conduisant ainsi à une double liaison entre C-6 et C-7.
R₇ représente :
- un atome d'hydrogène,
- un atome d'halogène,
- un groupe hydroxy, ORₓ, ou O-CORₓ dans lequel Rₓ est un atome d'hydrogène, un radical alkyle droit, ramifié ou cyclique contenant 1 à 8 atomes de carbone, un radical alkényle droit, ramifié ou cyclique contenant 2 à 8 atomes de carbone, un radical alkynyle droit ou ramifié contenant 3 à 8 atomes de carbone, ou un radical aryle, étant entendu que ces radicaux peuvent être éventuellement substitués, ou
- un groupe augmentant l'hydrosolubilité de formule générale -O-CO-A-COR_{y},
dans laquelle A est un radical alkényle, cycloalkényle ou aryle comportant au moins une double liaison,
R_{y} représente :
- un groupe hydroxyle,
- un groupe alkylamino, alkylaminoalkoxy, alkylaminoalkylthio ou N,N-dialkylaminocarbonoylalkoxy dans lequel le radical alkyle contient 1 à 4 atomes de carbone, éventuellement substitué, et
R₈ représente un radical méthyle ou forme un cyclopropyle avec R₇.

21. Utilisation des dérivés obtenus selon le procédé de l'une des revendications précédentes **caractérisé en ce que** pour la préparation du dérivé de la formule générale (VI) dans lequel R₂ représente une radical méthyle, R₆ représente un atome hydrogène, R₈ et R₇ forme un cyclopropyle, m est égal à 1, R représente un radical phényle et R₅ représente un radical méthyle, ou bien on fait réagir la baccatine III avec l'anhydride trifluorométhanesulfonique, puis avec un halogénure de métal alcalin ou avec un azoture de métal alcalin ou avec un sel d'ammonium quaternaire ou un phosphate de métal alcalin ou bien on fait réagir la baccatine III avec le DAST (diéthylaminosulfur trifluoride).

22. Utilisation des dérivés de formule générale (VI) obtenus selon l'une des revendications 20 ou 21 pour la préparation de taxoïdes thérapeutiquement actifs de formule générale (VII) : 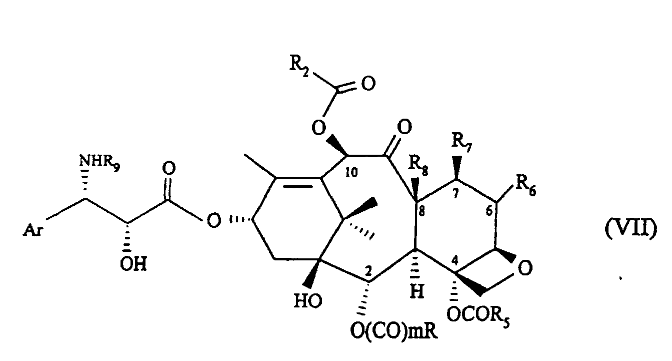 dans laquelle m est égal à 1,
R représente un radical phényle,
R₂, R₅ représentent un radical méthyle,
R₆ représente un atome d'hydrogène,
R₇ représente des substituents tels que définis dans la revendication 20,
R₈ peut représenter un radical méthyle ou former avec R₇ un cyclopropyle,
Ar représente un radical phényle,
R₉ représente un radical t-butoxy ou phényle.

23. Utilisation des dérivés de formule générale (VI) pour la préparation de taxoïdes tels que définis dans la revendication 22 pour lesquels m est égal à 1, R représente un radical phényle, R₂ et R₅ représententchacun un radical méthyle, R₆ représente un atome d'hydrogène, R₇ représente un radical hydroxy, Ar représente un radical phényle et R₉ représente un radical benzoyle ou bien m est égal à 1, R représente un radical phényle, R₂ et R₅ représentent chacun un radical méthyle, R₆ représente un atome d'hydrogène, R₈ et R₇ forment un cyclopropyle, Ar représente un radical phényle et R₉ représente un radical t-butoxy.

## Patentansprüche

1. Verfahren zur selektiven Monoacylierung von 10-Desacetyl-baccatin III oder seinen Derivaten in C-2 und C-4, die freie Hydroxylgruppen in den Positionen C-7 und C-10 besitzen, **dadurch gekennzeichnet, daß** man die Acylierung von 10-Desacetyl-baccatin III oder seinen Derivaten in C-2 und C-4 mit Hilfe eines Methylen-iminium-Salzes durchführt.

2. Verfahren nach Anspruch 1, **dadurch gekennzeichnet, daß** das Methylen-iminium-Salz der allgemeinen Formel (II) 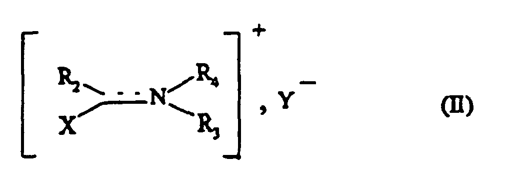 entspricht, in der R₂ darstellt:
- ein Wasserstoffatom,
- einen geraden, verzweigten oder cyclischen Rest Alkyl mit 1 bis 12 Kohlenstoffatomen,
- einen geraden, verzweigten oder cyclischen Rest Alkenyl mit 2 bis 12 Kohlenstoffatomen,
- einen geraden oder verzweigten Rest Alkinyl mit 3 bis 12 Kohlenstoffatomen,
- einen Rest Aryl,
- eine Gruppe Alkoxy, Alkylamino, Alkylthio, Alkyloxycarbonyl, Alkylaminocarbonyl, Alkylthiocarbonyl, bei denen jeder Teil Alkyl 1 bis 12 Kohlenstoffatome umfaßt, oder
- einen gesättigten oder ungesättigten heterocyclischen Rest mit 5 bis 6 Ringgliedern und einem oder mehreren Heteroatomen, ausgewählt unter Schwefel, Sauerstoff oder Stickstoff,
wobei diese Reste gegebenenfalls substituiert sind durch einen oder mehrere Substituenten, ausgewählt unter:
- den Halogenatomen,
- den Resten Alkyl oder Halogenalkyl,
- den Resten Aryl,
- den Gruppen Alkylamino, Piperidyl, Piperazinyl, Nitro oder Cyano,
- den Gruppen Alkoxy oder Alkoxycarbonyl, deren Teile Alkyl 1 bis 12 Kohlenstoffatome enthalten, und
- den gesättigten oder ungesättigten heterocyclischen Resten mit 5 bis 6 Ringgliedern und einem oder mehreren Heteroatomen, ausgewählt unter Schwefel, Sauerstoff oder Stickstoff, oder
R₂ und R₃ oder R₂ und R₄ zusammen mit dem Stickstoffatom und dem methylenischen Kohlenstoff einen Ring mit 4 bis 7 Ringgliedern bilden können,
R₃ und R₄, gleich oder verschieden, darstellen:
- ein Wasserstoffatom,
- einen geraden, verzweigten oder cyclischen Rest Alkyl mit 1 bis 12 Kohlenstoffatomen,
- einen geraden, verzweigten oder cyclischen Rest Alkenyl mit 2 bis 12 Kohlenstoffatomen,
- einen geraden oder verzweigten Rest Alkinyl mit 3 bis 12 Kohlenstoffatomen, oder
- einen Rest Aryl, oder
R₃ und R₄ zusammen mit dem Stickstoffatom einen gesättigten oder ungesättigten Ring mit 4 bis 6 Ringgliedern bilden können, gegebenenfalls substituiert,
X ein Halogenatom, eine Gruppe Phosphinyloxy, Phosphoranyloxy, Halogensulfinyloxy, Halogensulfonyloxy, Alkylsulfonyloxy, Arylsulfonyloxy, Alkylaminosulfonyloxy, Alkylcarbonyloxy, Arylcarbonyloxy, Halophosphinyloxy, Halophosphoranyloxy, Haloalkylsulfonyloxy, Haloalkylcarbonat,
Y⁻ ein Ion bedeutet, ausgewählt unter den Halogeniden, den Alkylsulfonaten, den Arylsulfonaten, den Alkylaminosulfonaten, den Alkylcarboxylaten, den Arylcarboxylaten, den Phosphorodihalogenidaten, oder [M(Z)ₙ]⁻, worin 4 ≤ n ≤ 6 ist, Z ein Halogenatom bedeutet und M ein Element des Periodensystems darstellt, das einen Oxidationsgrad von gleich oder höher 3, vorzugsweise zwischen 3 und 5 erreichen kann.

3. Verfahren nach Anspruch 2, **dadurch gekennzeichnet, daß** in der allgemeinen Formel (II)
X darstellt:
- ein Halogenatom,
- eine Gruppe (Hal)₄P-O-, (Hal)₂PO-O-, (Hal)SO-O-, (Hal)SO₂-O-, RₐSO₂-O- oder RₐCO₂, worin Rₐ einen linearen, verzweigten oder cyclischen Rest Alkyl, einen Rest Halogenalkyl oder einen Rest Aryl bedeutet, gegebenenfalls substituiert durch ein Halogenatom oder durch einen Rest Alkyl oder Nitro, und Hal ein Halogenatom ist, ausgewählt unter den Atomen von Fluor, Chlor, Brom oder Iod,
- Y⁻ darstellt:
ein Halogenid-Ion,
- ein Gegenion wie RₐCO₂⁻, RₐSO₃⁻ oder (Hal)₂PO₂⁻, worin Hal und Rₐ wie oben definiert sind, oder
- eine Einheit wie [M(Z)ₙ]⁻, worin 4 ≤ n ≤ 6 ist, Z ein Halogenatom bedeutet wie ein Atom von Fluor oder Chlor und M ein Element des Periodensystems darstellt, das einen Oxidationsgrad von gleich oder höher 3 haben kann, ausgewählt unter Aluminium, Bor, Antimon, Zinn und Titan,
R₂ Wasserstoff, ein Rest Methyl, Ethyl oder ein Rest Phenyl ist.

4. Verfahren nach einem der Ansprüche 1 bis 3, in dem die Iminium-Salze, die man verwendet, sind: das Trifluormethansulfonat von N,N-Dimethyl-trifluormethansulfonyloxy-1-methyliden-ammonium (R₂=H, R₃=R₄=CH₃, X=CF₃SO₃, Y⁻=CF₃SO₃⁻), das Trifluormethansulfonat von N-Methyl-trifluormethansulfonyloxy-1-ethyliden-ammonium (R₂=R₃=CH₃, R₄=H, X=CF₃SO₃, Y⁻=CF₃SO₃⁻), das Chlorid von N-Methylpara-toluolsulfonyloxy-1-ethyliden-ammonium (R₂=R₃=CH₃, R₄=H, X=p-CH₃-C₆H₄-SO₃, Y⁻=Cl⁻) oder das para-Toluolsulfonat von N-Methylchlor-1-ethyliden-ammonium (R₂=R₃=CH₃, R₄=H, X=Cl, Y⁻=p-CH₃-C₆H₄-SO₃⁻), das Chlorid von N-Methyl-chlor-1-ethylidenammonium (R₂=R₃=CH₃, R₄=H, X=Cl, Y⁻=Cl⁻), das Chlorid von N-Ethylmethansulfonyloxy-1-ethyliden-ammonium (R₂=CH₃, R₃=C₂H₅, R₄=H, X=CH₃SO₃, Y⁻=Cl⁻) oder das Methansulfonat von N-Ethyl-chlor-1-ethyliden-ammonium (R₂=CH₃, R₃=C₂H₅, R₄=H, X=Cl, Y⁻=CH₃SO₃⁻), das Chlorid von N-Ethyl-dichlorphosphinyloxy-1-ethyliden-ammonium (R₂=CH₃, R₃=C₂H₅, R₄=H, X=O-PO-Cl₂, Y⁻=Cl⁻) oder das Dichlorphosphoridat von N-Ethyl-chlor-1-ethyliden-ammonium (R₂=CH₃, R₃=C₂H₅, R₄=H, X=Cl, Y⁻=Cl₂PO₂⁻), das para-Toluolsulfonat von N,N-Diethyl-paratoluolsulfonyloxy-1-ethyliden-ammonium (R₂=CH₃, R₃=R₄=C₂H₅, X=p-CH₃-C₆H₄-SO₃), Y⁻=p-CH₃-C₆H₄-SO₃⁻).

5. Verfahren nach Anspruch 1, **dadurch gekennzeichnet, daß** das 10-Desacetyl-baccatin III und/oder seine Derivate in C-2 und C-4 der allgemeinen Formel (III) 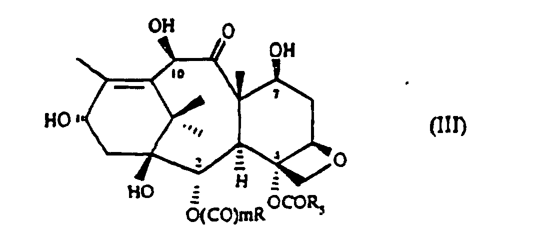 entspricht, in der m gleich 0 oder 1 ist,
R und R₅, gleich oder verschieden, darstellen:
- einen geraden, verzweigten oder cyclischen Rest Alkyl mit 1 bis 8 Kohlenstoffatomen,
- einen geraden, verzweigten oder cyclischen Rest Alkenyl mit 2 bis 8 Kohlenstoffatomen,
- einen geraden, verzweigten oder cyclischen Rest Alkinyl mit 3 bis 8 Kohlenstoffatomen,
- einen Rest Aryl, oder
- einen gesättigten oder ungesättigten heterocyclischen Rest mit 4 bis 6 Ringgliedern und einem oder mehreren Heteroatomen, ausgewählt unter den Atomen von Sauerstoff, Stickstoff oder Schwefel,
wobei diese Reste gegebenenfalls substituiert sind durch einen oder mehrere Substituenten, ausgewählt unter:
- den Halogenatomen,
- den Resten Alkyl, Hydroxy, Alkoxy, Alkylthio, Alkylamin, Halogenalkyl, Halogenalkoxy, Halogenalkylthio, deren Teil Alkyl 1 bis 4 Kohlenstoffatome enthält,
- den heterocyclischen Resten mit 4 bis 6 Ringgliedern, die ein oder mehrere Heteroatome umfassen, ausgewählt unter den Atomen von Sauerstoff, Stickstoff oder Schwefel, gegebenenfalls substituiert,
- den Resten Aryl, gegebenenfalls substituiert,
- den Gruppen Cyano, Nitro oder Azido, oder
- den Gruppen Carboxy oder Alkoxycarbonyl, deren Teil Alkyl 1 bis 4 Kohlenstoffatome enthält.

6. Verfahren nach Anspruch 5, **dadurch gekennzeichnet, daß** in der allgemeinen Formel (III) m gleich 1 ist, R einen Rest Phenyl darstellt und R₅ einen Rest Methyl bedeutet.

7. Verfahren nach Anspruch 1, **dadurch gekennzeichnet, daß** man ein wie in einem der Ansprüche 2 bis 5 definiertes Methylen-iminium-Salz mit einem wie in einem der Ansprüche 6 oder 7 definierten Derivat von 10-DAB zur Reaktion bringt, um ein Produkt der allgemeinen Formel (IV) 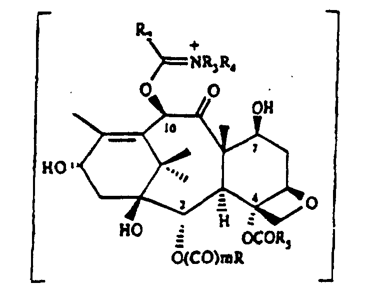 X⁻ oder Y⁻ (IV) zu erhalten, in der R₂, R₃, R₄, X⁻ und Y⁻ wie in einem der Ansprüche 2 bis 5 und m, R und R₅ wie in einem der Ansprüche 6 oder 7 definiert sind, das zu einem Produkt der allgemeinen Formel (V) 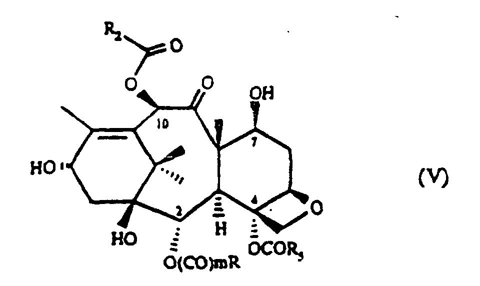 hydrolysiert wird, in der R₂ wie in einem der Ansprüche 2 bis 4 und m, R und R₅ wie in einem der Ansprüche 5 oder 6 definiert sind.

8. Verfahren nach einem der Ansprüche 1 bis 7, **dadurch gekennzeichnet, daß** man in einem organischen Lösungsmittel arbeitet.

9. Verfahren nach Anspruch 8, **dadurch gekennzeichnet, daß** das organische Lösungsmittel unter den halogenierten organischen Lösungsmitteln, den aromatischen Kohlenwasserstoffen, den aliphatischen Ethern, den aliphatischen Nitrilen oder den aliphatischen Amiden ausgewählt wird.

10. Verfahren nach Anspruch 8, **dadurch gekennzeichnet, daß** die halogenierten organischen Lösungsmittel unter den halogenierten aliphatischen Kohlenwasserstoffen wie Dichlormethan oder 1,2-Dichlorethan, die aromatischen Kohlenwasserstoffe unter Benzol, Toluol oder Xylol, die aliphatischen Ether unter Ethylether oder Isopropylether, die aliphatischen Nitrile unter Acetonitril oder die aliphatischen Amide unter N,N-Dimethylformamid, N,N-Dimethylacetamid, N-Ethylacetamid und N,N-Diethylformamid ausgewählt werden.

11. Verfahren nach irgendeinem der vorstehenden Ansprüche, **dadurch gekennzeichnet, daß** das Methylen-iminium-Salz der allgemeinen Formel (II) durch Kondensation des Amids der Formel 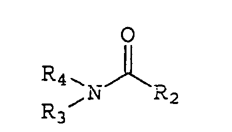 mit einem elektrophilen Reaktanden der Formel XY hergestellt wird.

12. Verfahren nach irgendeinem der Ansprüche 1 bis 10, **dadurch gekennzeichnet, daß** das Methylen-iminium-Salz in situ hergestellt oder vorher isoliert wird.

13. Verfahren nach Anspruch 1 bis 12, **dadurch gekennzeichnet, daß** man in Anwesenheit eines Überschusses an dem elektrophilen Reaktanden von unter oder gleich 10 Äquivalenten arbeitet, bezogen auf das Substrat der allgemeinen Formel (III).

14. Verfahren nach Anspruch 13, **dadurch gekennzeichnet, daß** der elektrophile Reaktand in einer Menge von etwa 5 Äquivalenten vorliegt, bezogen auf das Substrat der allgemeinen Formel (III).

15. Verfahren nach Anspruch 1, **dadurch gekennzeichnet, daß** die Reaktion der Methylen-iminium-Salze der allgemeinen Formel (II) mit dem Substrat der allgemeinen Formel (III) bei einer Temperatur zwischen -78 °C und der Rückflußtemperatur der Reaktionsmischung durchgeführt wird.

16. Verfahren nach Anspruch 15, **dadurch gekennzeichnet, daß** die Temperatur zwischen -20 °C und 25 °C liegt.

17. Verfahren nach Anspruch 7, **dadurch gekennzeichnet, daß** die Hydrolyse der Iminium-Salze der allgemeinen Formel (IV) mit Wasser oder mit Hilfe einer wäßrigen Lösung durchgeführt wird, die eine anorganische oder organische Base enthält.

18. Verfahren nach Anspruch 17, **dadurch gekennzeichnet, daß** die Base unter Natriumhydroxid, Pyridin, Triethylamin, Natriumacetat, Natriumcarbonat und Natriumhydrogencarbonat ausgewählt wird.

19. Neues Derivat von Baccatin III der allgemeinen Formel (IV) 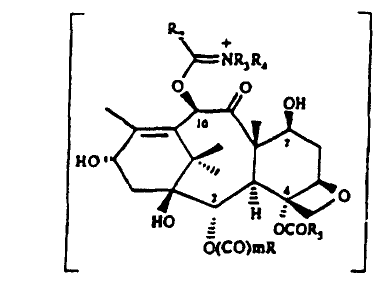 X⁻ oder Y⁻ (IV) in der R₂, R₃, R₄, X⁻ und Y⁻ wie in einem der Ansprüche 2 bis 5 und m, R und R₅ wie in einem der Ansprüche 6 oder 7 definiert sind.

20. Verwendung der nach irgendeinem der vorstehenden Ansprüche erhaltenen Produkte für die Herstellung von Analogen von Baccatin III der allgemeinen Formel (VI) 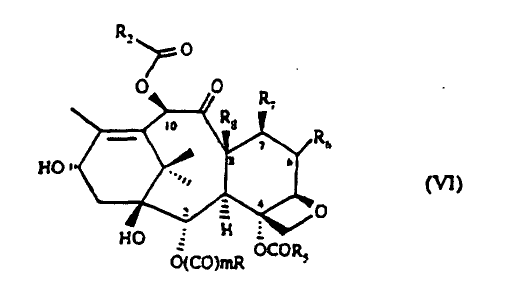 in der R₂ wie in einem der Ansprüche.2 bis 4 und m, R und R₅ wie in einem der Ansprüche 5 oder 6 definiert sind,
R₆ ein Wasserstoffatom ist oder eine Bindung mit R₇ bildet, was auf diese Weise zu einer Doppelbindung zwischen C-6 und C-7 führt,
R₇ darstellt:
- ein Wasserstoffatom,
- ein Halogenatom,
- eine Gruppe Hydroxy, ORₓ oder O-CORₓ, worin Rₓ ein Wasserstoffatom, einen geraden, verzweigten oder cyclischen Rest Alkyl mit 1 bis 8 Kohlenstoffatomen, einen geraden, verzweigten oder cyclischen Rest Alkenyl mit 2 bis 8 Kohlenstoffatomen, einen geraden oder verzweigten Rest Alkinyl mit 3 bis 8 Kohlenstoffatomen oder einen Rest Aryl, mit der Maßgabe, daß diese Reste gegebenenfalls substituiert sein können, oder
- eine die Wasserlöslichkeit steigernde Gruppe der allgemeinen Formel -O-CO-A-COR_{y}, in der A ein Rest Alkenyl, Cycloalkenyl oder Aryl ist, mindestens eine Doppelbindung umfassend,
R_{y} darstellt;
- eine Gruppe Hydroxyl,
- eine Gruppe Alkylamino, Alkylaminoalkoxy, Alkylaminoalkylthio oder N,N-Dialkylaminocarbonoylalkoxy, worin der Rest Alkyl 1 bis 4 Kohlenstoffatome enthält, gegebenenfalls substituiert, und
R₈ ein Rest Methyl ist oder mit R₇ ein Cyclopropyl bildet.

21. Verwendung der nach dem Verfahren von einem der vorstehenden Ansprüche erhaltenen Derivate, **dadurch gekennzeichnet, daß** man für die Herstellung des Derivates der allgemeinen Formel (VI), in der R₂ einen Rest Methyl darstellt, R₆ ein Wasserstoffatom ist, R₇ und R₈ ein Cyclopropyl bilden, m gleich 1 ist, R einen Rest Phenyl bedeutet und R₅ einen Rest Methyl darstellt, entweder das Baccatin III mit Trifluormethansulfonsäure-anhydrid und anschließend mit einem Alkalimetallhalogenid oder mit einem Alkalimetallazid oder mit einem quaternären Ammoniumsalz oder einem Alkalimetallphosphat zur Reaktion bringt, oder das Baccatin III mit DAST (Diethylaminosulfur-trifluorid) umsetzt.

22. Verwendung der nach einem der Ansprüche 20 oder 21 erhaltenen Derivate der allgemeinen Formel (VI) für die Herstellung von therapeutisch wirksamen Taxoiden der allgemeinen Formel (VII) 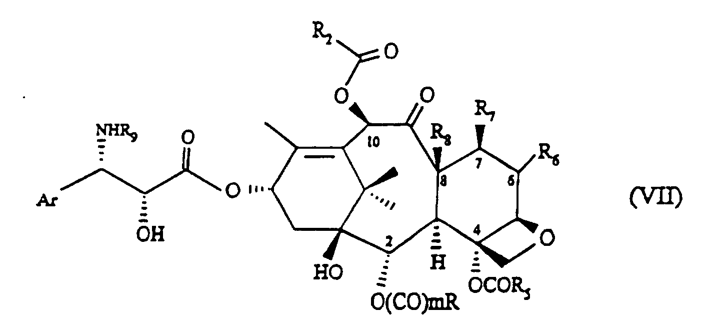 in der m gleich 1 ist,
R einen Rest Phenyl darstellt,
R₂, R₅ einen Rest Methyl darstellen,
R₆ ein Wasserstoffatom darstellt,
R₇ Substituenten darstellt, wie sie in Anspruch 20 definiert sind, R₈ einen Rest Methyl darstellen oder mit R₇ ein Cyclopropyl bilden kann,
Ar einen Rest Phenyl darstellt,
R₉ einen Rest tert.-Butoxy oder Phenyl darstellt.

23. Verwendung der Derivate der allgemeinen Formel (VI) für die Herstellung von Taxoiden wie in Anspruch 22 definiert, worin entweder m gleich 1 ist, R einen Rest Phenyl darstellt, R₂ und R₅ jeweils einen Rest Methyl bedeuten, R₆ ein Wasserstoffatom ist, R₇ einen Rest Hydroxy darstellt, Ar ein Rest Phenyl ist und R₉ einen Rest Benzoyl bedeutet, oder m gleich 1 ist, R einen Rest Phenyl darstellt, R₂ und R₅ jeweils einen Rest Methyl bedeuten, R₆ ein Wasserstoffatom ist, R₇ und R₈ ein Cyclopropyl bilden, Ar ein Rest Phenyl ist und R₉ einen Rest tert.-Butoxy bedeutet.

## Claims

1. Process for the selective monoacylation of 10-deacetylbaccatin III or derivatives thereof in positions C-2 and C-4, possessing free hydroxyl groups in positions C-7 and C-10, **characterized in that** 10-deacetylbaccatin III or derivatives thereof in positions C-2 and C-4 is acylated using a methyleneiminium salt.

2. Process according to Claim 1, **characterized in that** the methyleneiminium salt corresponds to the general formula (II) : 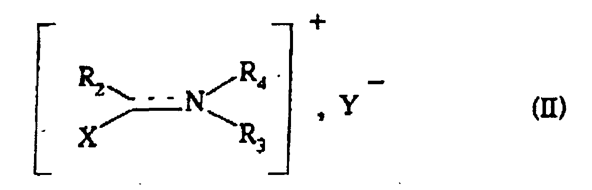 in which R₂ represents:
- a hydrogen atom,
- a straight, branched or cyclic alkyl radical containing 1 to 12 carbon atoms,
- a straight, branched or cyclic alkenyl radical containing 2 to 12 carbon atoms,
- a straight or branched alkynyl radical containing 3 to 12 carbon atoms,
- an aryl radical,
- an alkoxy, alkylamino, alkylthio, alkyloxycarbonyl, alkylaminocarbonyl or alkylthiocarbonyl group in which the alkyl part contains 1 to 12 carbon atoms, or
- a 5- or 6-membered saturated or unsaturated heterocyclic radical containing one or more hetero atoms chosen from sulphur, oxygen and nitrogen,
these radicals optionally being substituted with one or more substituents chosen from:
- halogen atoms,
- alkyl or haloalkyl radicals,
- aryl radicals,
- alkylamino, piperidyl, piperazinyl, nitro or cyano groups,
- alkoxy or alkoxycarbonyl groups,
the alkyl parts of the radicals containing 1 to 12 carbon atoms, and
- 5- or 6-membered saturated or unsaturated heterocyclic radicals containing one or more hetero atoms chosen from sulphur, oxygen and nitrogen, or alternatively
R₂ and R₃ or R₂ and R₄ may form, with the nitrogen atom and the methylenic carbon, a 4- to 7-membered ring,
R₃ and R₄, which are identical or different, represent:
- a hydrogen atom,
- a straight, branched or cyclic alkyl radical containing 1 to 12 carbon atoms,
- a straight, branched or cyclic alkenyl radical containing 2 to 12 carbon atoms,
- a straight or branched alkynyl radical containing 3 to 12 carbon atoms, or
- an aryl radical, or alternatively
R₃ and R₄ may form, with the nitrogen atom, an optionally substituted, saturated or unsaturated 4- to 6-membered ring,
X represents a halogen atom or a phosphinyloxy, phosphoranyloxy, halosulphinyloxy, halosulphonyloxy, alkylsulphonyloxy, arylsulphonyloxy, alkylaminosulphonyloxy, alkylcarbonyloxy, arylcarbonyloxy, halophosphinyloxy, halophosphoranyloxy, haloalkylsulphonyloxy or haloalkylcarbonate group,
Y⁻ represents an ion selected from halides, alkylsulphonates, arylsulphonates, alkylaminosulphonates, alkylcarboxylates, arylcarboxylates and phosphoro-dihalidates, or [M(Z)ₙ]⁻ in which 4 ≤ n ≤ 6, z is a halogen atom and M is an element from the Periodic Table which can have an oxidation state equal to or greater than 3 and preferably between 3 and 5.

3. Process according to Claim 2, **characterized in. that**, in the general formula (II), X represents:
- a halogen atom,
- a group (Hal)₄P-O-, (Hal)₂PO-O-, (Hal)SO-O-, (Hal)SO₂-O-, RₐSO₂-O- or RₐCO₂ in which Rₐ represents a linear, branched or cyclic alkyl radical, a haloalkyl radical, or an aryl radical optionally substituted with a halogen atom or with an alkyl or nitro radical, and Hal represents a halogen atom chosen from fluorine, chlorine, bromine and iodine atoms,
Y⁻ represents:
- a halide ion,
- a counterion such as RₐCO₂⁻, RₐSO₃⁻ or (Hal)₂PO₂⁻, in which Hal and Rₐ are defined as above, or
- a species such as [M(Z)ₙ]⁻ in which 4 ≤ n ≤ 6, Z is a halogen atom such as a fluorine or chlorine atom and M is an element from the Periodic Table which can have an oxidation state equal to or greater than 3, selected from aluminium, boron, antimony, tin and titanium.
R₂ represents hydrogen, a methyl or ethyl radical or a phenyl radical.

4. Process according to one of Claims 1 to 3, in which the iminium salts which are used are N,N-dimethyl-1-trifluoromethanesulphonyloxymethylideneammonium trifluoromethanesulphonate (R₂=H, R₃=R₄=CH₃, X=CF₃SO₃, Y⁻=CF₃SO₃⁻), N-methyl-1-trifluoromethanesulphonyloxyethylideneammonium trifluoromethanesulphonate (R₂=R₃=CH₃, R₄=H, X=CF₃SO₃, Y⁻=CF₃SO₃⁻), N-methyl-1-p-toluenesulphonyloxyethylideneammonium chloride (R₂=R₃=CH₃, R₄=H, X=p-CH₃-C₆H₄-SO₃, Y⁻=Cl⁻) or N-methyl-1-chloroethylideneammonium p-toluenesulphonate (R₂=R₃=CH₃, R₄=H, X=Cl, Y⁻=p-CH₃-C₆H₄-SO₃⁻), N-methyl-1-chloroethylideneammonium chloride (R₂=R₃=CH₃, R₄=H, X=Cl, Y⁻=Cl⁻), N-ethyl-1-methanesulphonyloxyethylideneammonium chloride (R₂=CH₃, R₃=C₂H₅, R₄=H, X=CH₃SO₃, Y⁻=Cl⁻) or N-ethyl-1-chloroethylideneammonium methanesulphonate (R₂=CH₃, R₃=C₂H₅, R₄=H, X=Cl, Y⁻=CH₃SO₃⁻), N-ethyl-1-dichlorophosphinyloxyethylideneammonium chloride, (R₂=CH₃, R₃=C₂H₅, R₄=H, X=O-PO-Cl₂, Y⁻=Cl⁻) or N-ethyl-1-chloroethylidene- ammonium dichlorophosphoridate, (R₂=CH₃, R₃=C₂H₅, R₄=H, X=Cl, Y⁻=Cl₂PO₂⁻), N,N-diethyl-1-p-toluenesulphonyloxy- ethylideneammonium p-toluenesulphonate (R₂=CH₃, R₃=R₄=C₂H₅, X=p-CH₃-C₆H₄-SO₃, Y⁻=p-CH₃-C₆H₄-SO₃⁻).

5. Process according to Claim 1, **characterized in that** the 10-deacetylbaccatin III and/or the derivatives thereof in positions C-2 and C-4 correspond to the general formula (III) : 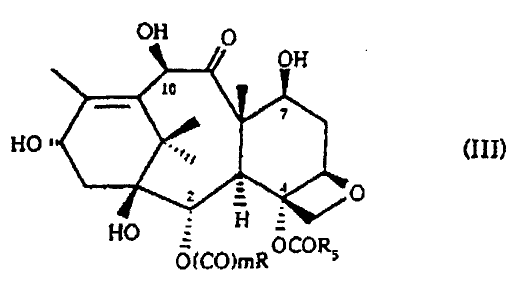 in which m is equal to 0 or 1,
R and R₅, which are identical or different, represent:
- a straight, branched or cyclic alkyl radical containing 1 to 8 carbon atoms,
- a straight, branched or cyclic alkenyl radical containing 2 to 8 carbon atoms,
- a straight, branched or cyclic alkynyl radical containing 3 to 8 carbon atoms,
- an aryl radical, or
- a 4- to 6-membered saturated or unsaturated heterocyclic radical containing one or more hetero atoms chosen from oxygen, nitrogen and sulphur atoms,
these radicals optionally being substituted with one or more substituents chosen from:
- halogen atoms,
- alkyl, hydroxyl, alkoxy, alkylthio, alkylamino, haloalkyl, haloalkoxy or haloalkylthio radicals in which the alkyl part contains 1 to 4 carbon atoms,
- 4- to 6-membered heterocyclic radicals containing one or more hetero atoms chosen from oxygen, nitrogen and sulphur atoms, which are optionally substituted,
- optionally substituted aryl radicals,
- cyano, nitro or azido groups, or
- carboxyl or alkoxycarbonyl groups in which the alkyl part contains 1 to 4 carbon atoms.

6. Process according to Claim 5, **characterized in that**, in the general formula (III), m is equal to 1, R represents a phenyl radical and R₅ represents a methyl radical.

7. Process according to Claim 1, **characterized in that** a methyleneiminium salt as defined in one of Claims 2 to 5 is reacted with a 10-DAB derivative as defined in either of Claims 6 and 7 in order to obtain a product of general formula (IV) : 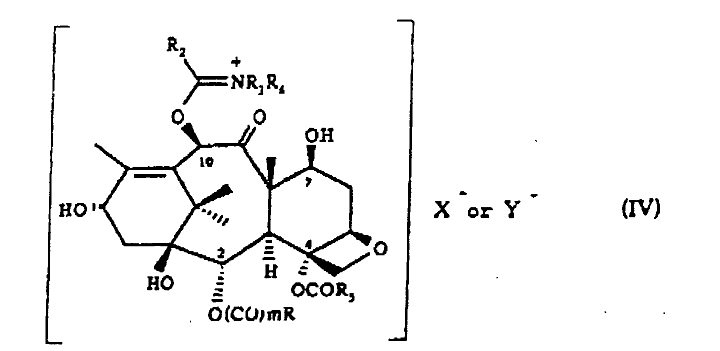 in which R₂, R₃, R₄, X⁻ and Y⁻ are defined as in one of Claims 2 to 5 and m, R and R₅ are defined as in either of Claims 6 and 7, which product is hydrolysed to a product of general formula (V): 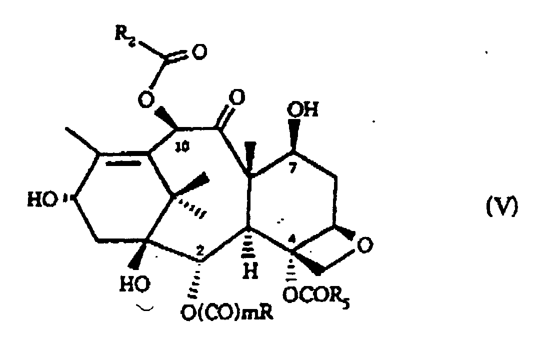 in which R₂ is defined as in one of Claims 2 to 4 and m, R and R₅ are defined as in either of Claims 5 and 6.

8. Process according to one of Claims 1 to 7, **characterized in that** it is carried out in an organic solvent.

9. Process according to Claim 8, **characterized in that** the organic solvent is chosen from halogenated organic solvents, aromatic hydrocarbons, aliphatic ethers, aliphatic nitriles and aliphatic amides.

10. Process according to Claim 8, **characterized in that** the halogenated organic solvents are chosen from chlorinated aliphatic hydrocarbons such as dichloromethane or 1,2-dichloroethane, the aromatic hydrocarbons are chosen from benzene, toluene and xylene, the aliphatic ethers are chosen from ethyl ether and isopropyl ether, aliphatic nitriles such as acetonitrile, or the aliphatic amides are chosen from N,N-dimethylformamide, N,N-dimethylacetamide, N-ethylacetamide and N,N-diethylacetamide.

11. Process according to any one of the preceding claims, **characterized in that** the methyleneiminium salt of formula (II) is prepared by condensation of the amide of formula 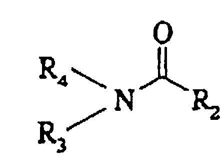 with an electrophilic reactant of formula XY.

12. Process according to any one of Claims 1 to 10, **characterized in that** the methyleneiminium salt is prepared in situ or isolated beforehand.

13. Process according to Claims 1 to 12, **characterized in that** it is carried out in the presence of an excess of electrophilic reactant of less than or equal to 10 equivalents relative to the substrate of general formula (III).

14. Process according to Claim 13, **characterized in that** the electrophilic reactant is in the region of 5 equivalents relative to the substrate of general formula (III).

15. Process according to Claim 1, **characterized in that** the reaction of the methyleneiminium salts of general formula (II) with the substrates of general formula (III) is carried out at a temperature of between -78°C and the reflux temperature of the reaction mixture.

16. Process according to Claim 15, **characterized in that** the temperature is between -20°C and 25°C.

17. Process according to Claim 7, **characterized in that** iminium salts of general formula (IV) are hydrolysed with water or using an aqueous solution containing an inorganic or organic base.

18. Process according to Claim 17, **characterized in that** the base is chosen from sodium hydroxide, pyridine, triethylamine, sodium acetate, sodium carbonate and sodium bicarbonate.

19. Novel baccatin III derivatives of general formula (IV) : 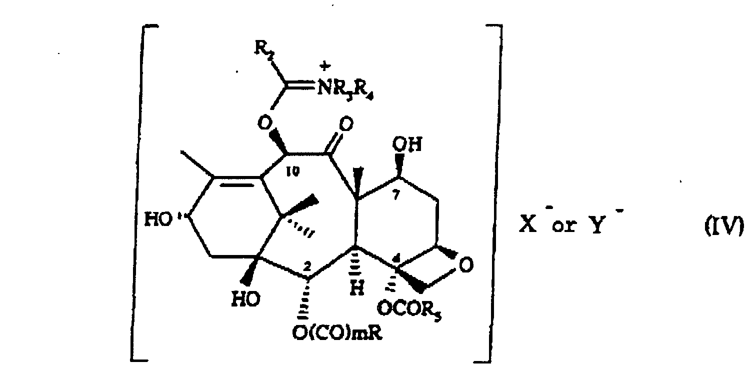 in which R₂, R₃, R₄, X⁻ and Y⁻ are defined as in one of Claims 2 to 5, and m, R and R₅ are defined as in either of Claims 6 and 7.

20. Use of the products obtained according to any one of the preceding claims for the preparation of baccatin III analogues of general formula (VI) : 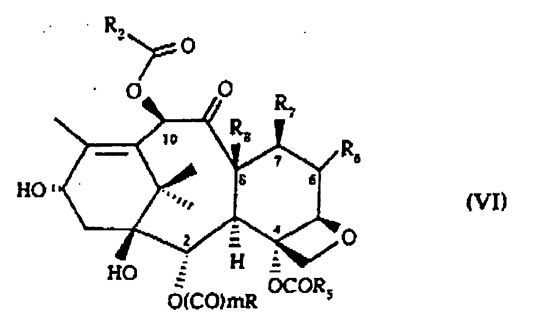 in which,
R₂ is defined as in one of Claims 2 to 4,
m, R and R₅ are defined as in either of Claims 5 and 6, R₆ represents a hydrogen atom, or forms a bond with R₇ thus leading to a double bond between C-6 and C-7,
R₇ represents:
- a hydrogen atom,
- a halogen atom,
- a hydroxyl, ORₓ or O-CORₓ group in which Rₓ is a hydrogen atom, a straight, branched or cyclic alkyl radical containing 1 to 8 carbon atoms, a straight, branched or cyclic alkenyl radical containing 2 to 8 carbon atoms, a straight or branched alkynyl radical containing 3 to 8 carbon atoms or an aryl radical, it being understood that these radicals may optionally be substituted, or
- a group which increases the water-solubility, of general formula -O-CO-A-COR_{y},
in which A is an alkenyl, cycloalkenyl or aryl radical containing at least one double bond,
R_{y} represents:
- a hydroxyl group,
- an optionally substituted alkylamino, alkylaminoalkyloxy, alkylaminoalkylthio or N,N-dialkylaminocarbonylalkoxy group in which the alkyl radical contains 1 to 4 carbon atoms,
and
R₈ represents a methyl radical or forms a cyclopropyl with R₇.

21. Use of the derivatives obtained according to the process of one of the preceding claims, **characterized in that**, for the preparation of the derivative of general formula (VI) in which R₂ represents a methyl radical, R₆ represents a hydrogen atom, R₈ and R₇ form a cyclopropyl, m is equal to 1, R represents a phenyl radical and R₅ represents a methyl radical, either by reacting baccatin III with trifluoromethanesulphonic anhydride and then with an alkali metal halide or with an alkali metal azide or with a quaternary ammonium salt or an alkali metal phosphate, or by reacting baccatin III with DAST (diethylaminosulphur trifluoride).

22. Use of the derivatives of general formula (VI) obtained according to either of Claims 20 and 21 for the preparation of therapeutically active taxoids of general formula (VII) : 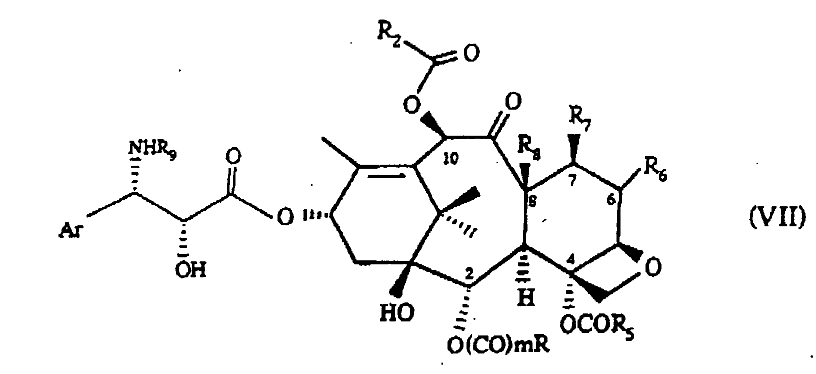 in which m is equal to 1,
R represents a phenyl radical,
R₂ and R₅ represent a methyl radical,
R₆ represents a hydrogen atom,
R₇ represents substituents as defined in Claim 20, R₈ may represent a methyl radical or form with R₇ a cyclopropyl,
Ar represents a phenyl radical, and
R₉ represents a t-butoxy or phenyl radical.

23. Use of the derivatives of general formula (VI) for the preparation of taxoids as defined in Claim 22 for which m is equal to 1, R represents a phenyl radical, R₂ and R₅ each represent a methyl radical, R₆ represents a hydrogen atom, R₇ represents a hydroxyl radical, Ar represents a phenyl radical and R₉ represents a benzoyl radical, or alternatively m is equal to 1, R represents a phenyl radical, R₂ and R₅ each represent a methyl radical, R₆ represents a hydrogen atom, R₈ and R₇ form a cyclopropyl, Ar represents a phenyl radical and R₉ represents a t-butoxy radical.
